(19) Europäisches Patentamt
European Patent Office
Office européen des brevets

(11) EP 0 568 096 B1

(12) EUROPEAN PATENT SPECIFICATION

(45) Date of publication and mention of the grant of the patent:
**29.07.1998 Bulletin 1998/31**

(51) Int Cl.6: **C07D 277/82**, A01N 47/36

(21) Application number: **93107070.0**

(22) Date of filing: **30.04.1993**

(54) **Benzothiazole derivative and agricultural and horticultural fingicide composition containing the same**

Benzothiazolederivate und diese enthaltende fungizide Zusammensetzungen für Landwirtschaft und Gartenbau

Dérivés de benzothiazole et compositions fongicides pour utilisation en agriculture et horticulture les contenant

(84) Designated Contracting States:
**DE ES FR GB IT NL PT**

(30) Priority: **30.04.1992 JP 135610/92**
**30.04.1992 JP 135611/92**

(43) Date of publication of application:
**03.11.1993 Bulletin 1993/44**

(73) Proprietor: **HODOGAYA CHEMICAL CO., LTD.**
**Tokyo (JP)**

(72) Inventors:
- **Tanaka, Ken-ichi**
  **Tsukuba-shi, Ibaraki (JP)**
- **Imai, Akihiro**
  **Tsukuba-shi, Ibaraki (JP)**
- **Matsuno, Shinichi**
  **Tsukuba-shi, Ibaraki (JP)**
- **Kasahara, Kaoru**
  **Tsukuba-shi, Ibaraki (JP)**
- **Kawada, Hiroshi**
  **Tsukuba-shi, Ibaraki (JP)**
- **Inayoshi, Chieko**
  **Tsukuba-shi, Ibaraki (JP)**

(74) Representative:
**Hansen, Bernd, Dr. Dipl.-Chem. et al**
**Hoffmann Eitle,**
**Patent- und Rechtsanwälte,**
**Postfach 81 04 20**
**81904 München (DE)**

(56) References cited:
**EP-A- 0 337 616**
**EP-A- 0 407 621**
**US-A- 3 671 531**

Remarks:
The file contains technical information submitted after the application was filed and not included in this specification

EP 0 568 096 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

Printed by Jouve, 75001 PARIS (FR)

## Description

### FIELD OF THE INVENTION

This invention relates to a novel benzothiazole derivative useful as an agricultural or horticultural fungicide and a fungicide composition containing the same.

### BACKGROUND OF THE INVENTION

Agriculture of today maintains high productivity owing to the use of fertilizers, agricultural chemicals, and various agricultural materials. On the other hand, the appearance of resistant microorganisms as a result of continuous application of agricultural chemicals and damages from agricultural chemicals as a result of repeated cultivation of the same crop on the same ground have given rise to great problems. Under these circumstances, development of chemicals of high safety has been keenly demanded.

As a fluorine-containing benzothiazole compound, fluorine-containing alkylbenzothiazole compounds represented by formula shown below are disclosed in U.S. Patent 3,671,531:

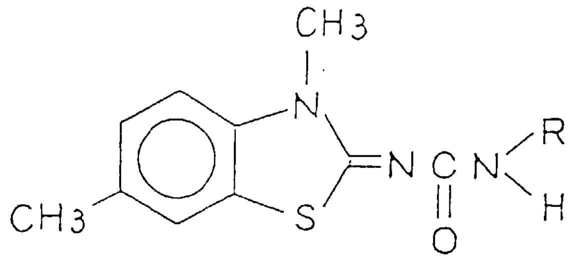

wherein R represents $CF_3$ or $CH_2CF_3$.
This prior art has no mention of the physical properties enough to specify the compounds. Neither is made reference to fungicidal activity of the compounds.

### SUMMARY OF THE INVENTION

An object of the present invention is to provide a novel fungicide compound having high safety.

As a result of extensive investigations, the present inventors have completed the present invention.

The present invention relates to a benzothiazole derivative represented by formula (I):

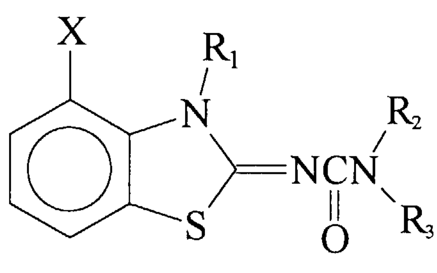

(I)

wherein $R_1$ represents an alkyl having from 1 to 8 carbon atoms, an alkenyl having from 2 to 6 carbon atoms, an alkynyl having from 2 to 6 carbon atoms, a cycloalkyl having from 3 to 6 carbon atoms, an alkoxyalkyl group having from 2 to 5 carbon atoms, a benzyl group, or an alkyl or alkoxyalkyl group having a carbonyl group and having from 2 to 5 carbon atoms; $R_2$ represents a hydrogen atom or an alkyl group having from 1 to 3 carbon atoms; $R_3$ represents an alkyl group having from 1 to 3 carbon atoms and containing from 1 to 6 fluorine atoms, or a group represented by $-O-R_4$ wherein $R_4$ represents a hydrogen atom, an alkyl group having from 1 to 8 carbon atoms, or a benzyl group; and X represents a halogen atom or a hydrogen atom.

The present invention also relates to an agricultural and horticultural fungicide composition comprising the benzothiazole derivative represented by formula (I) as an active ingredient.

DETAILED DESCRIPTION OF THE INVENTION

The alkyl group represented by $R_1$ in formula (I) contains from 1 to 8 carbon atoms, preferably from 1 to 6 carbon atoms. Specific examples thereof include methyl, ethyl, n-propyl, i-propyl, n-butyl, i-butyl, t-butyl, sec-butyl, cyclopropylmethyl, n-pentyl, i-pentyl, 2-methylbutyl, neopentyl, 1,1-diethylmethyl, 1-methylbutyl, t-pentyl, 1,2-dimethylpropyl, cyclobutylmethyl, cyclopropylethyl, 2-methylcyclopropylmethyl, n-hexyl, i-hexyl, 1-methylpentyl, 1,3-dimethylbutyl, 1,2,2-trimethylpropyl, 1-ethylbutyl, 1-ethyl-2-methylpropyl, cyclopentylmethyl, cyclobutylethyl, cyclopropylpropyl, 1,1,2-trimethylpropyl, 1,1-dimethylbutyl, 2,2-dimethylbutyl, 2-ethylbutyl, 2-methylpentyl, 2,3-dimethylbutyl, 3-methylpentyl, 3,3-dimethylbutyl, n-heptyl, 5-methylhexyl, 1-methylhexyl, 1,4-dimethylpentyl, 1,3-dimethylpentyl, 1,3,3-trimethylbutyl, 1-methyl-2-ethylbutyl, 2-methylhexyl, 2,4-dimethylpentyl, 2,3-dimethylpentyl, 1,1-dimethylpentyl, 2,2-dimethylpentyl, 3,3-dimethylpentyl, 4,4-diemthylpentyl, 3-methylhexyl, 3,4-dimethylpentyl, 4-methylhexyl, cyclohexylmethyl, cyclopentylethyl, cyclobutylpropyl, cyclopropylbutyl, n-octyl, 6-methylheptyl, 1-methylheptyl, 1,1-dimethylhexyl, 2-methylheptyl, 3-methylheptyl, 4-methylheptyl, 5-methylheptyl, 2,2-dimethylhexyl, 3,3-dimethylhexyl, 4,4-dimethylhexyl, 5,5-dimethylhexyl, cyclohexylethyl, cyclopentylpropyl, cylobutylbutyl and cyclopropylpentyl groups. Among them, methyl, ethyl, n-propyl, i-propyl, n-butyl, cyclopropylmethyl, n-hexyl and i-butyl groups are preferred.

The alkenyl group represented by $R_1$ in formula (I) contains from 2 to 6 carbon atoms, preferably from 3 to 4 carbon atoms. Specific examples thereof include vinyl, 1-propenyl, 2-propenyl, i-propenyl, 1-butenyl, 2-butenyl, 3-butenyl, 2-methyl-1-propenyl, 2-methyl-2-propenyl, 1-pentenyl, 3-pentenyl, 4-pentenyl, 1-methyl-3-butenyl, 1-methyl-2-butenyl, 1,2-dimethyl-2-propenyl, 1,1-dimethyl-2-propenyl, 1-hexenyl, 3-hexenyl, 4-hexenyl, 5-hexenyl, 1-methyl-2-pentenyl, 1-methyl-3-pentenyl, 1-methyl-4-pentenyl, 1,4-dimethyl-2-butenyl, 1,1-dimethyl-2-butenyl and 1,1-dimethyl-3-butenyl groups. Among them, 2-propenyl, 2-butenyl and 2-methyl-2-propenyl groups are preferred.

The alkynyl group represented by $R_1$ in formula (I) contains from 2 to 6 carbon atoms, preferably 3 carbon atoms. Specific examples thereof include ethynyl, 2-propynyl, 1-propynyl, 1-butynyl, 2-butynyl, 3-butynyl, 1-methyl-2-propynyl, 1-pentynyl, 2-pentynyl, 3-pentynyl, 4-pentynyl, 1-methyl-3-butynyl, 1-methyl-2-butynyl, 1,1-dimethyl-2-propynyl, 1-pentynyl, 2-hexynyl, 3-hexynyl, 4-hexynyl, 5-hexynyl, 1-methyl-2-pentynyl, 1-methyl-3-pentynyl, 1-methyl-4-hexynyl, 1,1-dimethyl-2-butynyl and 1,1-dimethyl-3-butynyl groups. Among them, 2-propynyl group is preferred.

The cycloalkyl group represented by $R_1$ in formula (I) contains from 3 to 6 carbon atoms, preferably 5 carbon atoms. Specific examples thereof include cyclopropyl, cyclobutyl, cyclopentyl and cyclohexyl groups. Among them, cyclopentyl group is preferred.

The alkoxyalkyl group represented by $R_1$ in formula (I) preferably contains from 2 to 5 carbon atoms, more preferably from 2 to 4 carbon atoms. Specific examples thereof include methoxymethyl, methoxyethyl, ethoxymethyl, propoxymethyl, ethoxyethyl, methoxypropyl, i-propoxymethyl, 1-methoxy-2-propyl, 2-methoxy-2-propyl, butoxymethyl, propoxyethyl, ethoxypropyl, methoxybutyl, i-butoxymethyl, t-butoxymethyl, sec-butoxymethyl, i-propoxyethyl and 2-ethoxy1-methylethyl groups.

The alkyl or alkoxyalkyl group containing a carbonyl group represented by $R_1$ in formula (I) preferably has one carbonyl group and contains from 2 to 5 carbon atoms, more preferably from 2 to 4 carbon atoms, in total. Specific examples thereof include carboxymethyl, acetylmethyl, methoxycarbonylmethyl, acetylethyl, propionylmethyl, methoxycarbonylethyl, ethoxycarbonylmethyl, carboxypropyl, acetylpropyl, propionylethyl, butyrylmethyl, i-butyrylmethyl, methoxycarbonylpropyl, propoxycarbonylmethyl, ethoxycarbonylethyl, carboxybutyl, 1-methoxycarbonyl-2-propyl groups.

Among the alkoxyalkyl groups and the alkyl or alkoxyalkyl groups containing a carbonyl group, carboxymethyl, methoxymethyl, methoxyethyl, acetylmethyl and ethoxycarbonylmethyl groups are preferred.

The alkyl group represented by $R_2$ in formula (I) contains from 1 to 3 carbon atoms, preferably 1 carbon atom. Specific examples thereof include methyl, ethyl, n-propyl and i-propyl groups. Among the groups represented by $R_2$, hydrogen atom and methyl group are preferred.

Specific examples of the $C_{1-3}$ alkyl group containing 1 to 6 fluorine atom represented by $R_3$ in formula (I) include trifluoromethyl, 2,2,2-trifluoroethyl, 2,2-difluoroethyl, 2-fluoroethyl, 1,2,2,2-tetrafluoroethyl, 1,1,2,2,2-pentafluoroethyl, 3,3,3-trifluoropropyl, 2,2,3,3,3-pentafluropropyl, 2,3,3,3-tetrafluoropropyl, 3-fluoropropyl, 3,3-difluoropropyl, 1,1,2,2,3,3-hexafluoropropyl, 1,2,2,3,3,3-hexafluoropropyl and 1,1,2,3,3,3-hexafluoropropyl groups. Among them, 2,2,2-trifluoroethyl, 2,2,3,3,3-pentafluoropropyl and 2-fluoroethyl groups are preferred.

The alkyl group represented by $R_4$ in formula (I) contains from 1 to 8 carbon atoms, preferably from 1 to 7 carbon atoms. Specific examples thereof include methyl, ethyl, n-propyl, i-propyl, n-butyl, i-butyl, t-butyl, sec-butyl, cyclopropylmethyl, n-pentyl, i-pentyl, 2-methylbutyl, neopentyl, 1,1-diethylmethyl, 1-methylbutyl, t-pentyl, 1,2-dimethylpropyl, cyclobutylmethyl, cyclopropylethyl, 2-methylcyclopropylmethyl, n-hexyl, i-hexyl, 1-methylpentyl, 1,3-dimethylbutyl, 1,2,2-trimethylpropyl, 1-ethylbutyl, 1-ethyl-2-methylpropyl, cyclopentylmethyl, cyclobutylethyl, cyclopropylpropyl, 1,1,2-trimethylpropyl, 1,1-dimethylbutyl, 2,2-dimethylbutyl, 2-ethylbutyl, 2-methylpentyl, 2,3-dimethylbutyl, 3-methylpentyl, 3,3-dimethylbutyl, n-heptyl, 5-methylhexyl, 1-methylhexyl, 1,4-dimethylpentyl, 1,3-dimethylpentyl, 1,3,3-trimethylbutyl, 1-methyl-2-ethylbutyl, 2-methylhexyl, 2,4-dimethylpentyl, 2,3-dimethylpentyl, 1,1-dimethylpentyl, 2,2-dimethylpentyl, 3,3-dimethylpentyl, 4,4-diemthylpentyl, 3-methylhexyl, 3,4-dimethylpentyl, 4-methylhexyl, cyclohexylmethyl, cyclopentylethyl, cyclobutylpropyl, cyclopropylbutyl, n-octyl, 6-methylheptyl, 1-methylheptyl, 1,4-dimethylpentyl, 2-methylheptyl, 3-methylheptyl, 4-methylheptyl, 5-methylheptyl, 2,2-dimethylhexyl, 3,3-dimethylhexyl, 4,4-dimethylhexyl, 5,5-dimethylhexyl, cyclohexylethyl, cyclopentylpropyl, cylobutylbutyl and cyclopropylpentyl groups. Among them, methyl, ethyl, n-propyl, i-propyl and n-butyl groups are preferred.

Examples of the halogen atom represented by X in formula (I) include fluorine, chlorine, bromine and iodine atoms. Among them, fluorine and chlorine atoms are preferred.

All the compounds included under formula (I) are novel compounds. They can be synthesized according to the following reaction scheme:

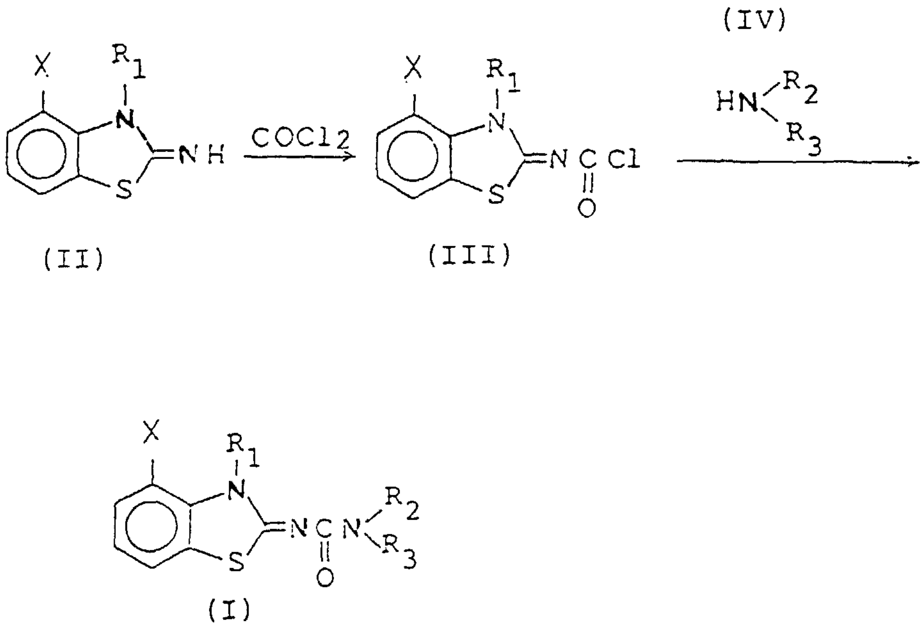


(IV)

(II)

(III)

(I)

wherein $R^1$, $R^2$, $R^3$, and X are as defined above.

The compound of formula (II) is reacted with phosgene which may be generated from trichloromethyl chloroformate, in a solvent, such as an aromatic hydrocarbon (e.g., benzene or toluene), a ketone (e.g., acetone or methyl isobutyl ketone), a halogenated hydrocarbon (e.g., chloroform or methylene chloride), an ester (e.g., methyl acetate or ethyl acetate), an ether (e.g., diethyl ether or diisopropyl ether) or a polar solvent (e.g., dioxane), preferably a halogenated hydrocarbon such as chloroform or methylene chloride, in the presence of a base (e.g., pyridine, triethylamine, sodium hydrogencarbonate or potassium hydrogencarbonate) at a temperature of from 0 to 30°C, preferably from 0 to 5°C to obtain the compound of formula (III). Without separating from the reaction mixture, the compound of formula (III) is then reacted with the compound of formula (IV) at a temperature of from 0 to 30°C, preferably from 0 to 5°C, to obtain the desired compound (I).

The starting compound (II) can be synthesized in accordance with known processes disclosed, e.g., in Organic Syntheses, Coll. Vol.3, p.76. The process disclosed in JP-A-2-202881 (the term "JP-A" as used herein means an "unexamined published Japanese patent application") is also employable.

The agricultural and horticultural fungicide composition according to the present invention contains the novel benzothiazole derivative of formula (I) as an active ingredient.

In applying the compound of formula (I) as a fungicide, the compound can be mixed with various carriers and formulated into various forms, such as wettable powders, emulsion, dusts, granules, and suspensions, in a usual manner.

Of the carriers, liquid carriers include conventional organic solvents, and solid carriers include conventional clay minerals and pumice powder. Surfactants may be used for improving emulsifiability, dispersibility, spreadability, etc. of the composition as hereinafter described in detail.

If desired, the compound may be used as admixture with or in combination with other agricultural chemicals such as herbicides, insecticides and other fungicides, or fertilizers.

The compound of the present invention should be applied in such an amount sufficient for obtaining desired effects. For example, the compound of formula (I) wherein $R_3$ is $-O-R_4$ may be applied in an amount of from 20 to 2,000 g/ha,

and the compound of formula (I) wherein $R_3$ is a $C_{1-3}$ alkyl group containing 1 to 6 fluorine atoms may be applied in an amount of from 50 to 2,000 g/ha. In general, a suitable amount of the compound to be applied ranges from 100 to 1,000 g/ha, which is applied in the form of a wettable powder, an emulsion, a dust, a granule, or a suspension containing the active ingredient in a concentration of from 0.1 to 50 % by weight.

Emulsions can be prepared by dissolving the active ingredient in an agriculturally acceptable organic solvent and adding a solvent-soluble emulsifying agent. Suitable solvents include xylene, o-chlorotoluene, cyclohexanone, isophorone, dimethylformamide, dimethyl sulfoxide, and a mixture thereof. An aromatic hydrocarbon or a mixture of an aromatic hydrocarbon, a ketone and a polar solvent is particularly preferred. A surfactant as an emulsifying agent is added in an amount of from 1 to 20% by weight based on the emulsion. Any of anionic, cationic or non-ionic surfactants may be employed. A suitable concentration of the active ingredient in the emulsion is from 0.5 to 50% by weight, preferably from 5 to 30% by weight, as for the compound of formula (I) wherein $R_3$ is $-O-R_4$; and from 0.5 to 20% by weight, preferably from 1 to 10% by weight, as for the compound of formula (I) wherein $R_3$ is a $C_{1-3}$ alkyl group containing 1 to 6 fluorine atoms.

Examples of suitable anionic surfactants include alkylsulfuric esters, alkyl diphenyl ether disulfonates, naphtylmethanesulfonates, lignin sulfonates, alkylsulfosuccinates, alkylbenzene sulfonates, and alkylphosphates. Examples of suitable cationic surfactants include alkylamine salts and quaternary ammonium salts. Examples of suitable nonionic surfactants include polyoxyethylene alkyl ethers, polyoxyethylene alkylaryl ethers, sorbitan fatty acid esters, polyoxyethylene sorbitan fatty acid esters, polyoxyethylene sorbitol fatty acid esters, glycerin fatty acid esters, and polyoxyethylene fatty acid esters.

Wettable powders can be prepared by adding the active ingredient to a finely pulverized inert solid carrier and a surfactant. Wettable powders generally contain from 2 to 50 % by weight of the active ingredient and from 1 to 20 % by weight of the surfactant. Suitable inert finely pulverized solid carriers include naturally-occurring clay, silicates, silica, and alkaline earth metal carbonates. Typical examples of such solid carriers are kaolin, zieclite, talc, diatomaceous earth, magnesium carbonate, calcium carbonate, and dolomite. Commonly employed anionic or nonionic surfactants (specific examples thereof have been described above) or mixtures thereof can be used as an emulsifying agent, a spreading agent or a dispersing agent.

Dusts can be prepared by adding the active ingredient to a commonly employed inert carrier, such as talc, a fine powder of clay, pyrophyllite, diatomaceous earth or magnesium carbonate, in a concentration ranging from 0.1 to 20% by weight, preferably from 0.5 to 5 % by weight.

Granules can be prepared by mixing the active ingredient with a finely pulverized inert carrier, such as bentonite, diatomaceous earth, kaolin, clay or talc, kneading the obtained mixture with water, and granulating by means of a granulating apparatus. Alternatively, granules may be prepared by adhering a solution of the active ingredient and a spreading agent onto a carrier having previously been granulated to a particle size of from about 15 to 30 mesh or a granular mineral, such as naturally-occurring pumice powder, acid clay or zeolite, having its particle size previously been regulated by grinding. Granules suitably contain the active ingredient in a concentration ranging from 0.2 to 20 % by weight, preferably from 1 to 10 % by weight.

Flowables can be prepared by finely pulverizing the active ingredient and mixing the resulting powder with a surfactants and water. Any of the above-mentioned anionic, cationic or nonionic surfactants can be used either individually or in combination thereof in an amount of from 1 to 20 % by weight. The content of the active ingredient in the flowable ranges from 1 to 50 % by weight, and preferably from 2 to 20 % by weight.

The compound of formula (I) is useful as an active ingredient of agricultural or horticultural fungicidal compositions and exhibits excellent controlling effects on rice blast, apple scab, pear scab, grape downy mildew, blue mold on various crops, powdery mildew or rust on various crops, and wheat leaf spot. The compound of the invention produces not only preventive effects but excellent curing effects and is therefore applicable for disease control after infection. The compound exhibits asmotransitional activity and is therefore applicable to soil for cauline and foliar disease control.

Typical examples of the compounds according to the present invention are shown in Table 1.

TABLE 1

| Compound No. | $-R_1$ | $-R_2$ | $-R_3$ | $-X$ | m.p. (°C) |
|---|---|---|---|---|---|
| 1 | $-CH_3$ | $-H$ | $-O-CH_3$ | $-H$ | 137-138 |
| 2 | $-CH_2CH_3$ | $-H$ | $-O-CH_3$ | $-H$ | 143-144 |
| 3 | $-CH_2CH_3$ | $-H$ | $-O-CH_2CH_3$ | $-H$ | 108-110 |
| 4 | $-n-C_3H_7$ | $-H$ | $-O-CH_3$ | $-H$ | 103-104 |
| 5 | $-CH(CH_3)_2$ | $-H$ | $-O-CH_3$ | $-H$ | 52-54 |
| 6 | $-CH_2CH=CH_2$ | $-H$ | $-O-CH_3$ | $-H$ | 110-111 |
| 7 | $-CH_2CH=CH_2$ | $-CH_3$ | $-O-CH_3$ | $-H$ | 62-63 |
| 8 | $-CH_2CH=CH_2$ | $-H$ | $-O-CH_2CH_3$ | $-H$ | 107-109 |
| 9 | $-CH_2CH=CH_2$ | $-H$ | $-O-n-C_3H_7$ | $-H$ | 80-81 |
| 10 | $-CH_2CH=CH_2$ | $-H$ | $-O-CH(CH_3)_2$ | $-H$ | 132-133 |
| 11 | $-CH_2CH=CH_2$ | $-H$ | $-O-n-C_4H_9$ | $-H$ | 56-57 |
| 12 | $-CH_2CH=CH_2$ | $-H$ | $-O-CH_2-C_6H_5$ | $-H$ | 85-87 |
| 13 | $-CH_2CH=CH_2$ | $-H$ | $-O-H$ | $-H$ | 184-185 |
| 14 | $-CH_2C\equiv CH$ | $-H$ | $-O-CH_3$ | $-H$ | 170-171 |
| 15 | $-CH_2(CH_2)_2CH_3$ | $-H$ | $-O-CH_3$ | $-H$ | 69-70 |
| 16 | $-CH_2CH(CH_3)_2$ | $-H$ | $-O-CH_3$ | $-H$ | 74-75 |
| 17 | $-CH_2-$ 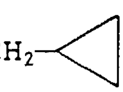 | $-H$ | $-O-CH_3$ | $-H$ | 109-110 |
| 18 | $-CH_2C=CH_2$<br>\|<br>$CH_3$ | $-H$ | $-O-CH_3$ | $-H$ | 93-94 |
| 19 | $-CH_2CH=CHCH_3$ | $-H$ | $-O-CH_3$ | $-H$ | 60-64 |

TABLE 1 (cont'd)

| Compound No. | $-R_1$ | $-R_2$ | $-R_3$ | $-X$ | m.p. (°C) |
|---|---|---|---|---|---|
| 20 | 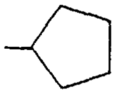 | $-H$ | $-O-CH_3$ | $-H$ | 45-48 |
| 21 | $-CH_2(CH_2)_4CH_3$ | $-H$ | $-O-CH_3$ | $-H$ | 66-68 |
| 22 | $-CH_2-C_6H_5$ | $-H$ | $-O-CH_3$ | $-H$ | 106-107 |
| 23 | $-CH_2COOC_2H_5$ | $-H$ | $-O-CH_3$ | $-H$ | 129-130 |
| 24 | $-CH_2CH_2OCH_3$ | $-H$ | $-O-CH_3$ | $-H$ | 124-125 |
| 25 | $-CH_2OCH_3$ | $-H$ | $-O-CH_3$ | $-H$ | 116-118 |
| 26 | $-CH_2CH=CH_2$ | $-H$ | $-O-CH_3$ | $-F$ | |
| 27 | $-CH_2CH=CH_2$ | $-H$ | $-O-CH_3$ | $-Cl$ | |
| 28 | $-CH_2-$ 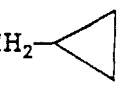 | $-H$ | $-CH_2CF_3$ | $-H$ | 141-142.5 |
| 29 | $-CH_2-C_6H_5$ | $-H$ | $-CH_2CF_3$ | $-H$ | 182-183 |
| 30 | $-CH_2CH(CH_3)-CH_3$ | $-H$ | $-CH_2CF_3$ | $-H$ | 140-141 |
| 31 | 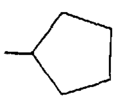 | $-H$ | $-CH_2CF_3$ | $-H$ | 165-166 |
| 32 | $-CH_2(CH_2)_4CH_3$ | $-H$ | $-CH_2CF_3$ | $-H$ | 117-118 |
| 33 | $-CH_2(CH_2)_2CH_3$ | $-H$ | $-CH_2CF_3$ | $-H$ | 110-111 |
| 34 | $-CH_2CH_2CH_3$ | $-H$ | $-CH_2CF_3$ | $-H$ | 135-136.5 |
| 35 | $-CH_2C(CH_3)=CH_2$ | $-H$ | $-CH_2CF_3$ | $-H$ | 125-126 |

TABLE 1 (cont'd)

| Compound No. | $-R_1$ | $-R_2$ | $-R_3$ | $-X$ | m.p. (°C) |
|---|---|---|---|---|---|
| 36 | $-CH(CH_3)-CH_3$ | $-H$ | $-CH_2CF_3$ | $-H$ | 159-160 |
| 37 | $-CH_2CH_3$ | $-H$ | $-CH_2CF_3$ | $-H$ | 129.5-130 |
| 38 | $-CH_2CH{=}CH_2$ | $-H$ | $-CH_2CF_3$ | $-H$ | 132.5-134.5 |
| 39 | $-CH_2C{\equiv}CH$ | $-H$ | $-CH_2CF_3$ | $-H$ | 167-168 |
| 40 | $-CH_2CH{=}CH_2$ | $-H$ | $-CH_2CF_2CF_3$ | $-H$ | 117-118 |
| 41 | $-CH_2CH{=}CH_2$ | $-H$ | $-CH_2CH_2F$ | $-H$ | 84-86 |
| 42 | $-CH_2COOC_2H_5$ | $-H$ | $-CH_2CF_3$ | $-H$ | 223-225 |
| 43 | $-CH_2COOH$ | $-H$ | $-CH_2CF_3$ | $-H$ | 198-201 |
| 44 | $-CH_2COCH_3$ | $-H$ | $-CH_2CF_3$ | $-H$ | 223 |
| 45 | $-CH_2CH_2OCH_3$ | $-H$ | $-CH_2CF_3$ | $-H$ | 144-146 |
| 46 | $-CH_2OCH_3$ | $-H$ | $-CH_2CF_3$ | $-H$ | 151-153 |
| 47 | $-CH_2CH{=}CH_2$ | $-H$ | $-CH_2CF_3$ | $-F$ | |
| 48 | $-CH_2CH{=}CH_2$ | $-H$ | $-CH_2CF_3$ | $-Cl$ | |

The present invention will now be illustrated in greater detail with reference to Synthesis Examples, Formulation Examples, and Test Examples, but it should be understood that the present invention is not construed as being limited thereto. All the percents and parts are by weight unless otherwise indicated.

SYNTHESIS EXAMPLE 1

Synthesis of 3-Methyl-2-N-methoxycarbamoyliminobenzothiazole (Compound No. 1)

In 100 mℓ of methylene chloride was dissolved 3.0 g of trichloromethyl chloroformate, and the solution was cooled to 0°C. To the solution was added dropwise a solution of 5.0 g of 3-methyl-2-iminobenzothiazole (the compound of formula (II) wherein $-R_1$ is $-CH_3$) and 2.4 g of pyridine in 50 mℓ of methylene chloride at 0 to 5°C, followed by stirring for 2 hours. To the mixture was added 4.2 g of O-methylhydroxylamine (the compound of formula (IV) wherein $-R_2$ is -H and $-R_3$ is $-O-CH_3$), followed by stirring at the same temperature for 4 hours. The reaction mixture was washed successively with diluted hydrochloric acid, a sodium hydrogencarbonate aqueous solution, water, and a saturated sodium chloride aqueous solution, dried over anhydrous magnesium sulfate, and distilled under reduced pressure to remove the solvent. The crude product thus obtained was purified by silica gel column chromatography to obtain 5.2 g (yield: 68 %) of the titled compound. Melting Point: 137-138°C.

SYNTHESIS EXAMPLE 2

Synthesis of 3-Allyl-2-N-methoxycarbamoyliminobenzothiazole (Compound No. 6)

In 100 mℓ of methylene chloride was dissolved 3.0 g of trichloromethyl chloroformate, and the solution was cooled to 0°C. To the solution was added dropwise a solution of 5.7 g of 3-allyl-2-iminobenzothiazole (the compound of formula (II) wherein $-R_1$ is $-CH_2CH=CH_2$) and 2.4 g of pyridine in 50 mℓ of methylene chloride at 0 to 5°C, followed by stirring for 2 hours. To the mixture was added 4.2 g of O-methylhydroxylamine (the compound of formula (IV) wherein $-R_2$ is -H and $-R_3$ is $-O-CH_3$), followed by stirring at the same temperature for 4 hours. The reaction mixture was washed successively with diluted hydrochloric acid, a sodium hydrogencarbonate aqueous solution, water, and a saturated sodium chloride aqueous solution, dried over anhydrous magnesium sulfate, and distilled under reduced pressure to remove the solvent. The crude product thus obtained was purified by silica gel column chromatography to obtain 4.8 g (yield: 61 %) of the titled compound. Melting Point: 110-111°C.

SYNTHESIS EXAMPLE 3

Synthesis of 3-Allyl-2-N-methyl-N-methoxycarbamoyliminobenzothiazole (Compound No. 7)

In 100 mℓ of methylene chloride was dissolved 3.0 g of trichloromethyl chloroformate, and the solution was cooled to 0°C. To the solution was added dropwise a solution of 5.7 g of 3-allyl-2-iminobenzothiazole (the compound of formula (II) wherein $-R_1$ is $-CH_2CH=CH_2$) and 2.4 g of pyridine in 50 mℓ of methylene chloride at 0 to 5°C, followed by stirring for 2 hours. To the mixture was added 4.2 g of N,O-dimethylhydroxylamine(the compound of formula (IV) wherein $-R_2$ is $-CH_3$ and $-R_3$ is $-CH_3$), followed by stirring at the same temperature for 4 hours. The reaction mixture was washed successively with diluted hydrochloric acid, a sodium hydrogencarbonate aqueous solution, water, and a saturated sodium chloride aqueous solution, dried over anhydrous magnesium sulfate, and distilled under reduced pressure to remove the solvent. The crude product thus obtained was purified by silica gel column chromatography to obtain 4.8 g (yield: 58 %) of the titled compound. Melting Point: 62-63°C.

SYNTHESIS EXAMPLE 4

Synthesis of 3-Allyl-2-N-n-propoxycarbamoyliminobenzothiazole (Compound No. 9)

In 100 mℓ of methylene chloride was dissolved 3.0 g of trichloromethyl chloroformate, and the solution was cooled to 0°C. To the solution was added dropwise a solution of 5.7 g of 3-allyl-2-iminobenzothiazole (the compound of formula (II) wherein $-R_1$ is $-CH_2CH=CH_2$) and 2.4 g of pyridine in 50 mℓ of methylene chloride at 0 to 5°C, followed by stirring for 2 hours. To the mixture was added 6.8 g of O-n-propylhydroxylamine (the compound of formula (IV) wherein $-R_2$ is -H and $-R_3$ is $-CH_2CH_2CH_3$), followed by stirring at the same temperature for 4 hours. The reaction mixture was washed successively with diluted hydrochloric acid, a sodium hydrogencarbonate aqueous solution, water, and a saturated sodium chloride aqueous solution, dried over anhydrous magnesium sulfate, and distilled under reduced pressure to remove the solvent. The crude product thus obtained was purified by silica gel column chromatography to obtain 4.7 g (yield: 57 %) of the titled compound. Melting Point: 80-81°C.

SYNTHESIS EXAMPLE 5

Synthesis of 3-Allyl-2-N-(2,2,2-trifluoroethyl)carbamoyliminobenzothiazole (Compound No. 38)

In 100 mℓ of methylene chloride was dissolved 3.0 g of trichloromethyl chloroformate, and the solution was cooled to 0°C. To the solution was added dropwise a solution of 5.7 g of 3-allyl-2-iminobenzothiazole and 2.4 g of pyridine in 50 mℓ of methylene chloride at 0 to 5°C, followed by stirring for 2 hours. To the mixture was added 8.9 g of 2,2,2-trifluoroethylamine, followed by stirring at the same temperature for 4 hours. The reaction mixture was washed successively with diluted hydrochloric acid, a sodium hydrogencarbonate aqueous solution, water, and a saturated sodium chloride aqueous solution, dried over anhydrous magnesium sulfate, and distilled under reduced pressure to remove the solvent. The crude product thus obtained was purified by silica gel column chromatography to obtain 5.6 g (yield: 59 %) of the titled compound. Melting Point: 132.5-134.5°C.

SYNTHESIS EXAMPLE 6

Synthesis of 3-Ethyl-2-N-(2,2,2-trifluoroethyl)carbamoyliminobenzothiazole (Compound No. 37)

In 100 mℓ of methylene chloride was dissolved 3.0 g of trichloromethyl chloroformate, and the solution was cooled to 0°C. To the solution was added dropwise a solution of 5.3 g of 3-ethyl-2-iminobenzothiazole and 2.4 g of pyridine in 50 mℓ of methylene chloride at 0 to 5°C, followed by stirring for 2 hours. To the mixture was added 8.9 g of 2,2,2-trifluoroethylamine, followed by stirring at the same temperature for 4 hours. The reaction mixture was washed successively with diluted hydrochloric acid, a sodium hydrogencarbonate aqueous solution, water, and a saturated sodium chloride aqueous solution, and dried over anhydrous magnesium sulfate. The solvent was removed by distillation under reduced pressure, and the crude product thus obtained was purified by silica gel column chromatography to obtain 5.6 g (yield: 62 %) of the titled compound. Melting Point: 129.5-130°C.

SYNTHESIS EXAMPLE 7

Synthesis of 3-Allyl-2-N-(2-fluoroethyl)carbamoyliminobenzothiazole (Compound No. 41)

In 100 mℓ of methylene chloride was dissolved 3.0 g of trichloromethyl chloroformate, and the solution was cooled to 0°C. To the solution was added dropwise a solution of 5.7 g of 3-allyl-2-iminobenzothiazole and 2.4 g of pyridine in 50 mℓ of methylene chloride at 0 to 5°C, followed by stirring for 2 hours. To the mixture was added 5.7 g of 2-fluoroethylamine, followed by stirring at the same temperature for 4 hours. The reaction mixture was washed successively with diluted hydrochloric acid, a sodium hydrogencarbonate aqueous solution, water, and a saturated sodium chloride aqueous solution, and dried over anhydrous magnesium sulfate. The solvent was removed by distillation under reduced pressure. The crude product thus obtained was purified by silica gel chromatography to obtain 5.4 g (yield: 64 %) of the titled compound. Melting Point: 84-86°C

FORMULATION EXAMPLE 1 (Emulsion)

| Component | Amount (part by weight) |
|---|---|
| Compound No. 10 | 9 |
| o-Chlorotoluene | 50 |
| Cyclohexanone | 36 |
| Sorpol 900B | 5 |

(Sorpol 900 B: a trademark of a product comprising a polyoxyethylenealkylphenol polymer, a polyoxyethylene alkylaryl ether, a polyoxyethylene sorbitan fatty acid ester and an anionic surfactant; manufactured by Toho Chemical Industry Co., Ltd.)

The above components were uniformly mixed and dissolved to obtain an emulsion according to the present invention.

FORMULATION EXAMPLE 2 (Wettable Powder)

| Component | Amount (part by weight) |
|---|---|
| Compound No. 3 | 25 |
| Kaolin clay | 63 |
| Sorpol 5039 | 6 |
| Sorpol 5060 | 6 |

(Sorpol 5039: a trademark of a product comprising an ammonium polyoxyethylene alkylaryl ether sulfate; Sorpol 5060: a trademark of a product comprising a sodium alkylallylsulfonate and phosphoric acid; each manufactured by Toho Chemical Industry Co., Ltd.)

The above components were mixed and ground to obtain a wettable powder according to the present invention.

| FORMULATION EXAMPLE 3 (Dust) | |
|---|---|
| Component | Amount (part by weight) |
| Compound No. 10 | 4 |
| Kaolin clay | 96 |

The above components were mixed and ground to obtain a dust according to the present invention.

| FORMULATION EXAMPLE 4 (Granule) | |
|---|---|
| Component | Amount (part by weight) |
| Compound No. 21 | 5 |
| Bentonite | 45 |
| Talc | 45 |
| Sodium lignin sulfonate | 5 |

The above components were uniformly mixed and ground, kneaded together with water, granulated, and dried to obtain a granule according to the present invention.

| FORMULATION EXAMPLE 5 (Flowable) | |
|---|---|
| Component | Amount (part by weight) |
| Compound No. 15 | 10 |
| Ethylene glycol | 5 |
| Sorpol 3078 | 5 |
| Sorpol 7512 | 1 |
| Water | 79 |
| (Sorpol 3078: a trademark of a product comprising a polyoxyethylene alkylphenyl ether sulfate; Sorpol 7512: a trademark of a product; each manufactured by Toho Chemical Industry Co., Ltd.) | |

The above components were uniformly mixed and ground to prepare a flowable according to the present invention.

| FORMULATION EXAMPLE 6 (Emulsion) | |
|---|---|
| Component | Amount (part by weight) |
| Compound No. 8 | 10 |
| Isophorone | 23 |
| o-Chlorotoluene | 28 |
| Xylene | 23 |
| Sorpol 900A | 8 |
| Sorpol 900B | 8 |
| (Sorpol 900 A: a trademark of a product comprising a polyoxyethylenealkylphenol polymer, a polyoxyethylene alkylaryl ether, a polyoxyethylene sorbitan fatty acid ester and an anionic surfactant; manufactured by Toho Chemical Industry Co., Ltd.) | |

The above components were uniformly mixed and dissolved to obtain an emulsion according to the present invention.

| FORMULATION EXAMPLE 7 (Emulsion) | |
|---|---|
| Component | Amount (part by weight) |
| Compound No. 28 | 10 |
| o-Chlorotoluene | 50 |
| Cyclohexanone | 36 |

(continued)

| FORMULATION EXAMPLE 7 (Emulsion) | |
|---|---|
| Component | Amount (part by weight) |
| Sorpol 900B | 4 |

The above components were uniformly mixed and dissolved to prepare an emulsion according to the present invention.

| FORMULATION EXAMPLE 8 (Wettable Powder) | |
|---|---|
| Component | Amount (part by weight) |
| Compound No. 30 | 30 |
| Kaolin clay | 60 |
| Sorpol 5039 | 5 |
| Sorpol 5060 | 5 |

The above components were mixed and ground to obtain a wettable powder according to the present invention.

| FORMULATION EXAMPLE 9 (Dust) | |
|---|---|
| Component | Amount (part by weight) |
| Compound No. 37 | 5 |
| Kaolin clay | 95 |

The above components were mixed and ground to obtain a dust according to the present invention.

| FORMULATION EXAMPLE 10 (Granule) | |
|---|---|
| Component | Amount (part by weight) |
| Compound No. 32 | 5 |
| Bentonite | 45 |
| Talc | 45 |
| Sodium lignin sulfonate | 5 |

The above components were uniformly mixed and ground, kneaded together with water, granulated, and dried to obtain a granule according to the present invention.

| FORMULATION EXAMPLE 11 (Flowable) | |
|---|---|
| Component | Amount (part by weight) |
| Compound No. 33 | 10 |
| Ethylene glycol | 5 |
| Sorpol 3078 | 5 |
| Sorpol 7512 | 1 |
| Water | 79 |

The above components were uniformly mixed and ground to prepare a flowable according to the present invention.

| FORMULATION EXAMPLE 12 (Emulsion) | |
|---|---|
| Component | Amount (part by weight) |
| Compound No. 30 | 10 |
| Isophorone | 23 |

(continued)

| FORMULATION EXAMPLE 12 (Emulsion) | |
|---|---|
| Component | Amount (part by weight) |
| o-Chlorotoluene | 28 |
| Xylene | 23 |
| Sorpol 900A | 8 |
| Sorpol 900B | 8 |

The above components were uniformly mixed and dissolved to obtain an emulsion according to the present invention.

TEST EXAMPLE 1

Preventive Effect Against Wheat Powdery Mildew:

Wheat plants (variety: *Norin No.* 61) were cultivated in a plastic pot having a diameter of 7 cm. At the difoliate stage, 10 mℓ of an emulsion of a test compound shown in Table 2 below prepared in accordance with Formulation Example 1 and adjusted to a concentration of 500 ppm was sprayed over the plant using a spray gun. Regarding each test compound, four lots each having 13 plants were employed, and two of them were treated with the test compound (test lot) and the other two of them were not treated with the test compound (control lot). One day after the treatment, conidia of *Erysiphe graminis* which causes wheat powdery mildew were inoculated by spraying. The plants were cultivated at 20°C in the dark for 12 hours followed by at 20°C for 6 days with 12-hour photoperiod. On the 7th day after the inoculation, the lesions of the first and second leaves were examined, and the preventive value was calculated in accordance with the following Equation (1).

Equation (1): $$\text{Prevention value} = \frac{(\text{Rate of lesion spot of control lot}) - (\text{Rate of lesion spot of test lot})}{(\text{Rate lesion spot of control lot})} \times 100$$

The results obtained are shown in Table 2.

TABLE 2

| Compound No. | Preventive Value |
|---|---|
| 1 | 100 |
| 2 | 100 |
| 3 | 100 |
| 4 | 100 |
| 5 | 100 |
| 6 | 100 |
| 7 | 100 |
| 8 | 100 |
| 9 | 100 |
| 10 | 100 |
| 11 | 100 |
| 12 | 100 |
| 13 | 100 |
| 14 | 100 |
| 15 | 100 |
| 16 | 100 |
| 17 | 100 |
| 18 | 100 |
| 19 | 100 |
| 20 | 100 |

TABLE 2 (continued)

| Compound No. | Preventive Value |
|---|---|
| 21 | 100 |
| 22 | 100 |
| 23 | 100 |
| 24 | 100 |
| 25 | 100 |
| 26 | 100 |
| 27 | 100 |

TEST EXAMPLE 2

Preventive Effect Against Rice Blast:

Rice (variety: *Koshihikari*) was cultivated in a plastic pot having a diameter of 7 cm. At the difoliate stage, 10 mℓ of a wettable powder of a test compound shown in Table 3 below prepared in accordance with Formulation Example 2 and adjusted to a concentration of 500 ppm was sprayed over the plant using a spray gun. Regarding each test compound, four lots each having 10 plants were employed, and two of them were treated with the test compound (test lot), and the other two of them were not treated with the test compound (control lot). One day after the treatment, a conidium suspension of *Pyricularia oryzae* which causes rice blast was inoculated. The plants were cultivated under moisture-saturation at 25°C in the dark for 48 hours followed by at 25°C for 6 days with 12-hour photoperiod. On the 7th day after the inoculation, the lesions of the first and second leaves were examined, and a preventive value was calculated in the same manner as in Test Example 1. The results obtained are shown in Table 3.

TABLE 3

| Compound No. | Preventive Value |
|---|---|
| 1 | 100 |
| 2 | 100 |
| 3 | 100 |
| 4 | 100 |
| 5 | 100 |
| 6 | 100 |
| 7 | 100 |
| 8 | 100 |
| 9 | 100 |
| 10 | 100 |
| 11 | 100 |
| 12 | 100 |
| 13 | 100 |
| 14 | 100 |
| 15 | 100 |
| 16 | 100 |
| 17 | 100 |
| 18 | 100 |
| 19 | 100 |
| 20 | 100 |
| 21 | 100 |
| 22 | 100 |
| 23 | 100 |
| 24 | 100 |
| 25 | 100 |
| 26 | 100 |

TABLE 3 (continued)

| Compound No. | Preventive Value |
|---|---|
| 27 | 100 |

TEST EXAMPLE 3

Preventive Effect Against Wheat Powdery Mildew:

Wheat (species: *Norin No.* 61) was cultivated in a plastic pot having a diameter of 7 cm. At the difoliate stage, 10 mℓ of an emulsifiable concentrate of a test compound shown in Table 4 below prepared in accordance with Formulation Example 7 and adjusted to a concentration of 500 ppm was sprayed over the plant using a spray gun. Regarding each test compound, four lots each having 13 plants were employed, and two of them were treated with the test compound (test lot) and the other two of them were not treated with the test compound (control lot). One day after the treatment, conidia of *Erysiphe graminis* which causes wheat powdery mildew were inoculated by spraying. The plants were cultivated at 20°C in the dark for 12 hours followed by at 20°C for 6 days with 12-hour photoperiod. On the 7th day after the inoculation, the lesions of the first and second leaves were examined, and the preventive value was calculated in the same manner as in Test Example 1.

TABLE 4

| Compound No. | Preventive Value |
|---|---|
| 28 | 100 |
| 29 | 100 |
| 30 | 100 |
| 31 | 100 |
| 32 | 100 |
| 33 | 100 |
| 34 | 100 |
| 35 | 100 |
| 36 | 100 |
| 37 | 100 |
| 38 | 100 |
| 39 | 100 |
| 40 | 100 |
| 41 | 100 |
| 42 | 100 |
| 43 | 100 |
| 44 | 100 |
| 45 | 100 |
| 46 | 100 |
| 47 | 100 |
| 48 | 100 |

TEST EXAMPLE 4

Preventive Effect Against Rice Blast:

Rice (species: *Koshihikari*) was cultivated in a plastic pot having a diameter of 7 cm. At the difoliate stage, 10 mℓ of a wettable powder of a test compound shown in Table 6 below prepared in accordance with Formulation Example 8 and adjusted to a concentration of 500 ppm was sprayed over the plant using a spray gun. Regarding each test compound, four lots each having 10 plants were employed, and two of them were treated with the test compound (test lot), and the other two of them were not treated with the test compound (control lot). One day after the treatment, a conidium suspension of *Pyricularia oryzae* which causes rice blast was inoculated. The plants were cultivated under moisture-saturation at 25°C in the dark for 48 hours followed by at 25°C for 6 days with 12-hour photoperiod. On the 7th day after the inoculation, the lesions of the first and second leaves were examined, and a preventive value was

calculated in the same manner as in Test Example 1. The results obtained are shown in Table 5.

TABLE 5

| Compound No. | Preventive Value |
|---|---|
| 28 | 100 |
| 29 | 100 |
| 30 | 100 |
| 31 | 100 |
| 32 | 100 |
| 33 | 100 |
| 34 | 100 |
| 35 | 100 |
| 36 | 100 |
| 37 | 100 |
| 38 | 100 |
| 39 | 100 |
| 40 | 100 |
| 41 | 100 |
| 42 | 100 |
| 43 | 100 |
| 44 | 100 |
| 45 | 100 |
| 46 | 100 |
| 47 | 100 |
| 48 | 100 |

The present invention provides novel compounds which, as formulated into various forms, exhibit excellent effects on various plant diseases and are useful as agricultural and horticultural fungicides.

While the invention has been described in detail and with reference to specific examples thereof, it will be apparent to one skilled in the art that various changes and modifications can be made therein without departing from the spirit and scope thereof.

**Claims**

**1.** A benzothiazole derivative represented by formula (I):

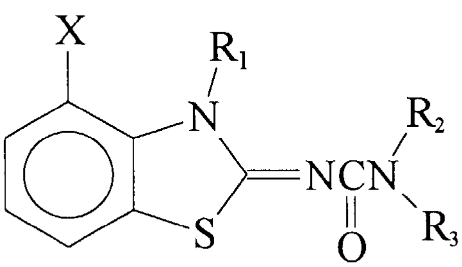

(I)

wherein $R_1$ represents an alkyl having from 1 to 8 carbon atoms, an alkenyl having from 2 to 6 carbon atoms, an alkynyl having from 2 to 6 carbon atoms, a cycloalkyl having from 3 to 6 carbon atoms, an alkoxyalkyl group having from 2 to 5 carbon atoms, a benzyl group, or an alkyl or alkoxyalkyl group having a carbonyl group and having from 2 to 5 carbon atoms; $R_2$ represents a hydrogen atom or an alkyl group having from 1 to 3 carbon atoms; $R_3$ represents an alkyl group having from 1 to 3 carbon atoms and containing from 1 to 6 fluorine atoms, or a group represented by -O-$R_4$ wherein $R_4$ represents a hydrogen atom, an alkyl group having from 1 to 8 carbon atoms,

or a benzyl group; and X represents a halogen atom or a hydrogen atom.

2. The benzothiazole derivative of claim 1, which is selected from 3-methyl-2-N-methoxycarbamoyliminobenzothiazole, 3-ethyl-2-N-methoxycarbamoyliminobenzothiazole, 3-ethyl-2-N-ethoxycarbamoyliminobenzothiazole, 3-n-propyl-2-N-methoxycarbamoyliminobenzothiazole, 3-i-propyl-2-N-methoxycarbamoyliminobenzothiazole, 3-allyl-2-N-methoxycarbamoyliminobenzothiazole, 3-allyl-2-N-methyl-N-methoxycarbamoyliminobenzothiazole, 3-allyl-2-N-ethoxycarbamoyliminobenzothiazole, 3-allyl-2-N-n-propoxycarbamoyliminobenzothiazole, 3-allyl-2-N-(2-propoxy)carbamoyliminobenzothiazole, 3-allyl-2-N-n-butoxycarbamoyliminobenzothiazole, 3-allyl-2-N-benzyloxycarbamoyliminobenzothiazole, 3-allyl-2-N-hydroxycarbamoyliminobenzothiazole, 3-propynyl-2-N-methoxycarbamoyliminobenzothiazole, 3-n-butyl-2-N-methoxycarbamoyliminobenzothiazole, 3-i-butyl-2-N-methoxycarbamoyliminobenzothiazole, 3-cyclopropylmethyl-2-N-methoxycarbamoyliminobenzothiazole, 3-(2-methyl-2-propenyl)-2-N-methoxycarbamoyliminobenzothiazole, 3-(2-butenyl)-2-N-methoxycarbamoyliminobenzothiazole, 3-cyclopentyl-2-N-methoxycarbamoyliminobenzothiazole, 3-n-hexyl-2-N-methoxycarbamoyliminobenzothiazole, 3-benzyl-2-N-methoxycarbamoyliminobenzothiazole, 3-ethoxycarbonylmethyl-2-N-methoxycarbamoyliminobenzothiazole, 3-methoxyethyl-2-N-methoxycarbamoyliminobenzothiazole, 3-methoxymethyl-2-N-methoxycarbamoyliminobenzothiazole, 3-allyl-4-fluoro-2-N-methoxycarbamoyliminobenzothiazole, 3-allyl-4-chloro-2-N-methoxycarbamoyliminobenzothiazole, 3-cyclopropylmethyl-2-N-(2,2,2-trifluoroethyl)carbamoyliminobenzothiazole, 3-benzyl-2-N-(2,2,2-trifluoroethyl)carbamoyliminobenzothiazole, 3-i-butyl-2-N-(2,2,2-trifluoroethyl)-carbamoyliminobenzothiazole, 3-cyclopentyl-2-N-(2,2,2-trifluoroethyl)carbamoyliminobenzothiazole, 3-n-hexyl-2-N-(2,2,2-trifluoroethyl)carbamoyliminobenzothiazole, 3-n-butyl-2-N-(2,2,2-trifluoroethyl)carbamoyliminobenzothiazole, 3-n-propyl-2-N-(2,2,2-trifluoroethyl)carbamoyliminobenzothiazole, 3-(2-methyl-2-propenyl)-2-N-(2,2,2-trifluoroethyl)carbamoyliminobenzothiazole, 3-i-propyl-2-N-(2,2,2-trifluoroethyl)-carbamoyliminobenzothiazole, 3-ethyl-2-N-(2,2,2-trifluoroethyl)carbamoyliminobenzothiazole, 3-allyl-2-N-(2,2,2-trifluoroethyl)carbamoyliminobenzothiazole, 3-(2-propynyl)-2-N-(2,2,2-trifluoroethyl)carbamoyliminobenzothiazole, 3-allyl-2-N-(2,2,3,3,3-pentafluoropropyl)carbamoyliminobenzothiazole, 3-allyl-2-N-(2-fluoroethyl)carbamoyliminobenzothiazole, 3-ethoxycarbonylmethyl-2-N-(2,2,2-trifluoroethyl)carbamoyliminobenzothiazole, 3-carboxymethyl-2-N-(2,2,2-trifluoroethyl)carbamoyliminobenzothiazole, 3-acetylmethyl-2-N-(2,2,2-trifluoroethyl)carbamoyliminobenzothiazole, 3-methoxyethyl-2-N-(2,2,2-trifluoroethyl)carbamoyliminobenzothiazole, 3-methoxymethyl-2-N-(2,2,2-trifluoroethyl)carbamoyliminobenzothiazole, 3-allyl-4-fluoro-2-N-(2,2,2-trifluoroethyl)-carbamoyliminobenzothiazole, and 3-allyl-4-chloro-2-N-(2,2,2-trifluoroethyl)carbamoyliminobenzothiazole.

3. A fungicide composition comprising a benzothiazole derivative represented by formula (I):

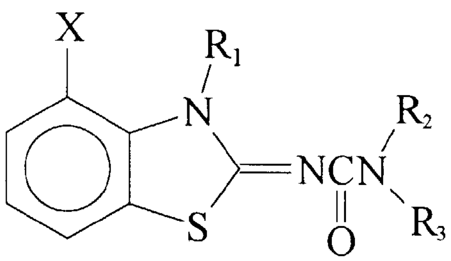

(I)

wherein $R_1$ represents an alkyl having from 1 to 8 carbon atoms, an alkenyl having from 2 to 6 carbon atoms, an alkynyl having from 2 to 6 carbon atoms, a cycloalkyl having from 3 to 6 carbon atoms, an alkoxyalkyl group having from 2 to 5 carbon atoms, a benzyl group, or an alkyl or alkoxyalkyl group having a carbonyl group and having from 2 to 5 carbon atoms; $R_2$ represents a hydrogen atom or an alkyl group having from 1 to 3 carbon atoms; $R_3$ represents an alkyl group having from 1 to 3 carbon atoms and containing from 1 to 6 fluorine atoms, or a group represented by -O-$R_4$ wherein $R_4$ represents a hydrogen atom, an alkyl group having from 1 to 8 carbon atoms, or a benzyl group; and X represents a halogen atom or a hydrogen atom; and a carrier.

4. A process for preparing a benzothiazole derivative represented by the formula (I):

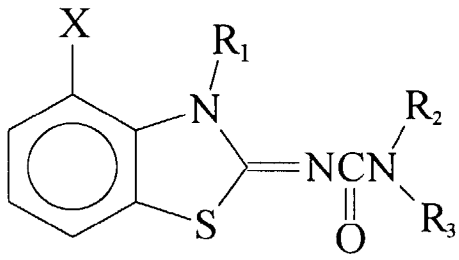

(I)

wherein $R_1$ represents an alkyl having from 1 to 8 carbon atoms, an alkenyl having from 2 to 6 carbon atoms, an alkynyl having from 2 to 6 carbon atoms, a cycloalkyl having from 3 to 6 carbon atoms, an alkoxyalkyl group having from 2 to 5 carbon atoms, a benzyl group, or an alkyl or alkoxyalkyl group having a carbonyl group and having from 2 to 5 carbon atoms; $R_2$ represents a hydrogen atom or an alkyl group having from 1 to 3 carbon atoms; $R_3$ represents an alkyl group having from 1 to 3 carbon atoms and containing from 1 to 6 fluorine atoms, or a group represented by $-O-R_4$ wherein $R_4$ represents a hydrogen atom, an alkyl group having from 1 to 8 carbon atoms, or a benzyl group; and X represents a halogen atom or a hydrogen atom,
which comprises the following steps:

(a) A compound represented by the following formula (II):

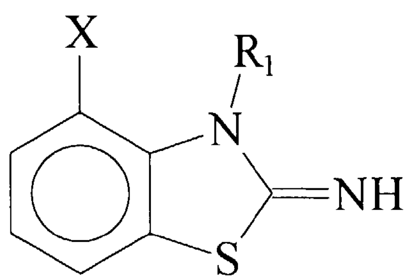

(II)

is reacted with phosgene in a suitable solvent selected from the group consisting of an aromatic hydrocarbon, a ketone, a halogenated hydrocarbon, an ester, an ether and a polar organic solvent such as dioxane, in the presence of a base at a temperature from 0 to 30°C to obtain a compound of the following formula (III):

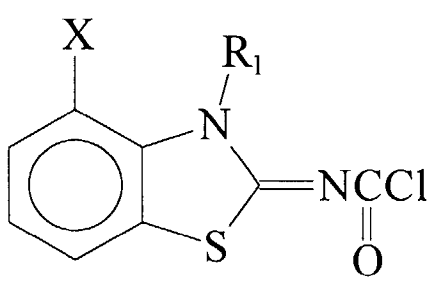

(III)

(b) The compound according to the general formula (III) is then reacted with an amine compound of the following formula (IV):

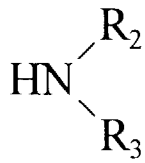

(IV)

at a temperature of from 0 to 30°C to obtain the desired compound (I).

5. A process according to claim 4 wherein in step (a) the solvent is a halogenated hydrocarbon, preferably chloroform or methylene chloride, the base is selected from pyridine, triethylamine, sodium hydrogencarbonate or potassium hydrogencarbonate, and the reaction temperature is from 0 to 5°C, and in step (b), the reaction temperature is from 0 to 5°C.

6. A process for preparing a fungicide composition comprising a benzothiazole derivative represented by formula (I):

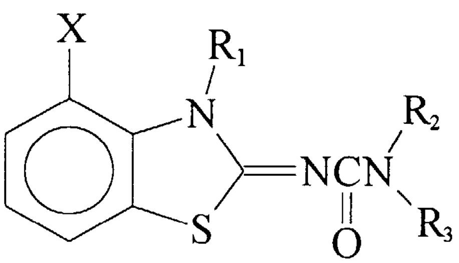

(I)

wherein $R_1$ represents an alkyl having from 1 to 8 carbon atoms, an alkenyl having from 2 to 6 carbon atoms, an alkynyl having from 2 to 6 carbon atoms, a cycloalkyl having from 3 to 6 carbon atoms, an alkoxyalkyl group having from 2 to 5 carbon atoms, a benzyl group, or an alkyl or alkoxyalkyl group having a carbonyl group and having from 2 to 5 carbon atoms; $R_2$ represents a hydrogen atom or an alkyl group having from 1 to 3 carbon atoms; $R_3$ represents an alkyl group having from 1 to 3 carbon atoms and containing from 1 to 6 fluorine atoms, or a group represented by $-O-R_4$ wherein $R_4$ represents a hydrogen atom, an alkyl group having from 1 to 8 carbon atoms, or a benzyl group; and X represents a halogen atom or a hydrogen atom; wherein said derivative of formula (I) is brought into contact with a suitable carrier in order to obtain a fungicidal composition containing the active ingredient in a concentration of from 0.1 to 50% by weight.

7. The use of a benzothiazole derivative represented by the formula (I):

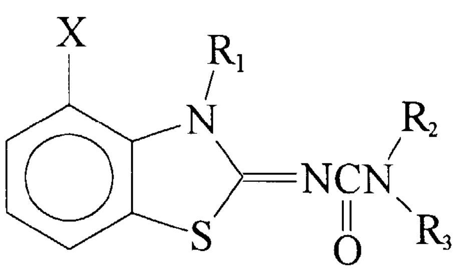

(I)

wherein $R_1$ represents an alkyl having from 1 to 8 carbon atoms, an alkenyl having from 2 to 6 carbon atoms, an alkynyl having from 2 to 6 carbon atoms, a cycloalkyl having from 3 to 6 carbon atoms, an alkoxyalkyl group having from 2 to 5 carbon atoms, a benzyl group, or an alkyl or alkoxyalkyl group having a carbonyl group and having from 2 to 5 carbon atoms; $R_2$ represents a hydrogen atom or an alkyl group having from 1 to 3 carbon atoms; $R_3$ represents an alkyl group having from 1 to 3 carbon atoms and containing from 1 to 6 fluorine atoms, or a group

represented by -O-$R_4$ wherein $R_4$ represents a hydrogen atom, an alkyl group having from 1 to 8 carbon atoms, or a benzyl group; and X represents a halogen atom or a hydrogen atom, as active ingredient in an agricultural or horticultural fungicidal composition.

**8.** The use according to claim 7 wherein the fungicidal composition is effective against wheat powdery mildew and rice blast.

**Patentansprüche**

**1.** Benzothiazol-Derivat, dargestellt durch die Formel (I):

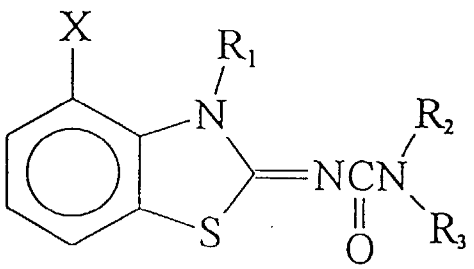

(I)

worin $R_1$ Alkyl, mit 1 bis 8 Kohlenstoffatomen, Alkenyl mit 2 bis 6 Kohlenstoffatomen, Alkinyl mit 2 bis 6 Kohlenstoffatomen, Cycloalkyl mit 3 bis 6 Kohlenstoffatomen, eine Alkoxyalkylgruppe mit 2 bis 5 Kohlenstoffatomen, eine Benzylgruppe oder eine Alkyl- oder Alkoxyalkylgruppe mit einer Carbonylgruppe und mit 2 bis 5 Kohlenstoffatomen darstellt; $R_2$ ein Wasserstoffatom oder eine Alkylgruppe mit 1 bis 3 Kohlenstoffatomen darstellt; $R_3$ eine Alkylgruppe mit 1 bis 3 Kohlenstoffatomen, die 1 bis 6 Fluoratome enthält, oder eine Gruppe -O-$R_4$ darstellt, worin $R_4$ ein Wasserstoffatom, eine Alkylgruppe mit 1 bis 8 Kohlenstoffatomen, oder eine Benzylgruppe ist; und X ein Halogenatom oder ein Wasserstoffatom ist.

**2.** Benzothiazol-Derivat nach Anspruch 1, ausgewählt aus:
3-Methyl-2-N-methoxycarbamoyliminobenzothiazol, 3-Ethyl-2-N-methoxycarbamoyliminobenzothiazol, 3-Ethyl-2-N-ethoxycarbamoyliminobenzothiazol, 3-n-Propyl-2-N-methoxycarbamoyliminobenzothiazol, 3-i-Propyl-2-N-methoxycarbamoyliminobenzothiazol, 3-Allyl-2-N-methoxycarbamoyliminobenzothiazol, 3-Allyl-2-N-methyl-N-methoxycarbamoyliminobenzothiazol, 3-Allyl-2-N-ethoxycarbamoyliminobenzothiazol, 3-Allyl-2-N-n-propoxycarbamoyliminobenzothiazol, 3-Allyl-2-N-(2-propoxy)carbamoyliminobenzothiazol, 3-Allyl-2-N-n-butoxycarbamoyliminobenzothiazol, 3-Allyl-2-N-benzyloxycarbamoyliminobenzothiazol, 3-Allyl-2-N-hydroxycarbamoyliminobenzothiazol, 3-Propinyl-2-N-methoxycarbamoyliminobenzothiazol, 3-n-Butyl-2-N-methoxycarbamoyliminobenzothiazol, 3-i-Butyl-2-N-methoxycarbamoyliminobenzothiazol, 3-Cyclopropylmethyl-2-N-methoxycarbamoyliminobenzothiazol, 3-(2-Methyl-2-propenyl)-2-N-methoxycarbamoyliminobenzothiazol, 3-(2-Butenyl)-2-N-methoxycarbamoyliminobenzothiazol, 3-Cyclopentyl-2-N-methoxycarbamoyliminobenzothiazol, 3-n-Hexyl-2-N-methoxycarbamoyliminobenzothiazol, 3-Benzyl-2-N-methoxycarbamoyliminobenzothiazol, 3-Ethoxycarbonylmethyl-2-N-methoxycarbamoyliminobenzothiazol, 3-Methoxyethyl-2-N-methoxycarbamoyliminobenzothiazol, 3-Methoxymethyl-2-N-methoxycarbamoyliminobenzothiazol, 3-Allyl-4-fluoro-2-N-methoxycarbamoyliminobenzothiazol, 3-Allyl-4-chloro-2-N-methoxycarbamoyliminobenzothiazol, 3-Cyclopropylmethyl-2-N-(2,2,2-trifluorethyl)carbamoyliminobenzothiazol, 3-Benzyl-2-N-(2,2,2-trifluorethyl)carbamoyliminobenzothiazol, 3-i-Butyl-2-N-(2,2,2-trifluorethyl) carbamoyliminobenzothiazol, 3-Cyclopentyl-2-N-(2,2,2-trifluorethyl)carbamoyliminobenzothiazol, 3-n-Hexyl-2-N-(2,2,2-trifluorethyl)carbamoyliminobenzothiazol, 3-n-Butyl-2-N-(2,2,2-trifluorethyl)carbamoyliminobenzothiazol, 3-n-Propyl-2-N-(2,2,2-trifluorethyl)carbamoyliminobenzothiazol, 3- (2-Methyl-2-propenyl)-2-N-(2,2,2-trifluorethyl) carbamoyliminobenzothiazol, 3-i-Propyl-2-N-(2,2,2-trifluorethyl)carbamoyliminobenzothiazol, 3-Ethyl-2-N-(2,2,2-trifluorethyl)carbamoyliminobenzothiazol, 3-Allyl-2-N-(2,2,2-trifluorethyl)carbamoyliminobenzothiazol, 3-(2-Propinyl)-2-N-(2,2,2-trifluorethyl)carbamoyliminobenzothiazol, 3-Allyl-2-N-(2,2,3,3,3-pentafluoropropyl)carbamoyliminobenzothiazol, 3-Allyl-2-N-(2-fluorethyl)carbamoyliminobenzothiazol, 3-Ethoxycarbonylmethyl-2-N-(2,2,2-trifluorethyl)carbamoyliminobenzothiazol, 3-Carboxymethyl-2-N-(2,2,2-trifluorethyl)carbamoyliminobenzothiazol, 3-Acetylmethyl-2-N-(2,2,2-trifluorethyl)carbamoyliminobenzothiazol, 3-Methoxyethyl-2-N-(2,2,2-trifluorethyl)carbamoyliminobenzothiazol, 3-Methoxymethyl-2-N-(2,2,2-trifluorethyl)carbamoyliminobenzothiazol, 3-Allyl-4-fluoro-2-N-(2,2,2-trifluorethyl)carbamoyliminobenzothiazol und 3-Allyl-4-chloro-2-N-(2,2,2-trifluorethyl)carbamoyliminobenzothiazol.

**3.** Fungizid-Zusammensetzung, umfassend ein durch Formel (I)

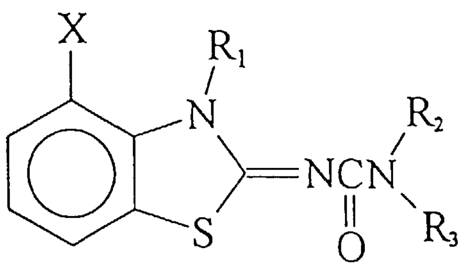

(I)

dargestelltes Benzothiazol-Derivat,
worin $R_1$ Alkyl mit 1 bis 8 Kohlenstoffatomen, Alkenyl mit 2 bis 6 Kohlenstoffatomen, Alkinyl mit 2 bis 6 Kohlenstoffatomen, Cycloalkyl mit 3 bis 6 Kohlenstoffatomen, eine Alkoxyalkylgruppe mit 2 bis 5 Kohlenstoffatomen, eine Benzylgruppe oder eine Alkyl- oder Alkoxyalkylgruppe mit einer Carbonylgruppe und mit 2 bis 5 Kohlenstoffatomen darstellt; $R_2$ ein Wasserstoffatom oder eine Alkylgruppe mit 1 bis 3 Kohlenstoffatomen darstellt; $R_3$ eine Alkylgruppe mit 1 bis 3 Kohlenstoffatomen, die 1 bis 6 Fluoratome enthält, oder eine Gruppe -O-$R_4$ darstellt, worin $R_4$ ein Wasserstoffatom, eine Alkylgruppe mit 1 bis 8 Kohlenstoffatomen, oder eine Benzylgruppe ist; und X ein Halogenatom oder ein Wasserstoffatom darstellt; und einen Träger.

**4.** Verfahren zum Herstellen eines durch die Formel (I)

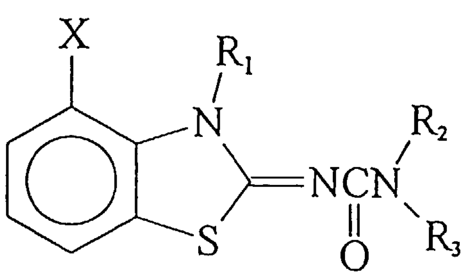

(I)

dargestellten Benzothiazol-Derivats,
worin $R_1$ Alkyl mit 1 bis 8 Kohlenstoffatomen, Alkenyl mit 2 bis 6 Kohlenstoffatomen, Alkinyl mit 2 bis 6 Kohlenstoffatomen, Cycloalkyl mit 3 bis 6 Kohlenstoffatomen, eine Alkoxyalkylgruppe mit 2 bis 5 Kohlenstoffatomen, eine Benzylgruppe oder eine Alkyl- oder Alkoxyalkylgruppe mit einer Carbonylgruppe und mit 2 bis 5 Kohlenstoffatomen darstellt; $R_2$ ein Wasserstoffatom oder eine Alkylgruppe mit 1 bis 3 Kohlenstoffatomen darstellt; $R_3$ eine Alkylgruppe mit 1 bis 3 Kohlenstoffatomen, die 1 bis 6 Fluoratome enthält, oder eine Gruppe -O-$R_4$ darstellt, worin $R_4$ ein Wasserstoffatom, eine Alkylgruppe mit 1 bis 8 Kohlenstoffatomen, oder eine Benzylgruppe ist; und X ein Halogenatom oder ein Wasserstoffatom darstellt,
das die folgenden Schritte umfaßt:

(a) Eine durch die folgende Formel (II)

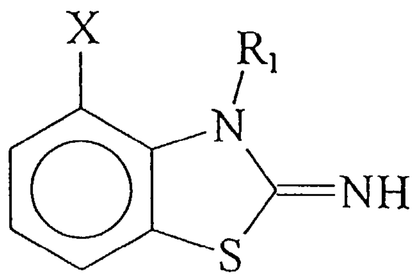

(II)

dargestellte Verbindung wird mit Phosgen in einem geeigneten Lösungsmittel ausgewählt aus der Gruppe bestehend aus einem aromatischen Kohlenwasserstoff, einem Keton, einem halogenierten Kohlenwasserstoff, einem Ester, einem Ether und einem polaren organischen Lösungsmittel, wie etwa Dioxan, in der Gegenwart einer Base bei einer Temperatur von 0 bis 30 °C umgesetzt, so daß man eine Verbindung der folgenden Gleichung (III)

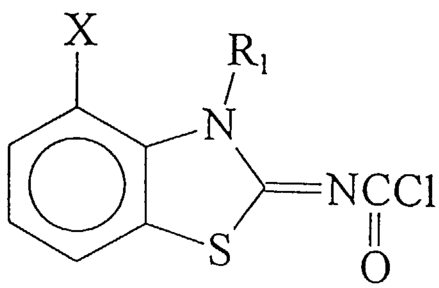

(III)

erhält.

(b) Die Verbindung gemäß der allgemeinen Formel (III) wird dann mit einer Aminverbindung der folgenden Formel (IV)

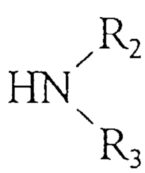

(IV)

bei einer Temperatur von 0 bis 30 °C umgesetzt, so daß man die gewünschte Verbindung (I) erhält.

**5.** Verfahren nach Anspruch 4, worin im Schritt (a) das Lösungsmittel ein halogenierter Kohlenwasserstoff, vorzugsweise Chloroform oder Methylenchlorid, ist, die Base aus Pyridin, Triethylamin, Natriumhydrogencarbonat oder Kaliumhydrogencarbonat ausgewählt ist, und die Reaktionstemperatur 0 bis 5 °C beträgt, und in Schritt (b) die Temperatur 0 bis 5 °C ist.

**6.** Verfahren zum Herstellen einer Fungizid-Zusammensetzung, die ein durch Formel (I)

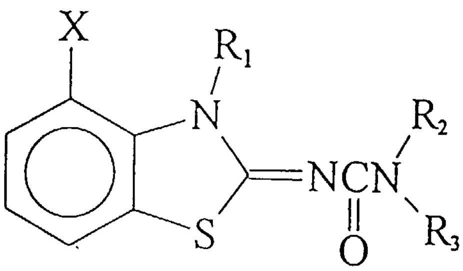

(I)

dargestelltes Benzothiazol-Derivat umfaßt,
worin $R_1$ Alkyl mit 1 bis 8 Kohlenstoffatomen, Alkenyl mit 2 bis 6 Kohlenstoffatomen, Alkinyl mit 2 bis 6 Kohlenstoffatomen, Cycloalkyl mit 3 bis 6 Kohlenstoffatomen, eine Alkoxyalkylgruppe mit 2 bis 5 Kohlenstoffatomen, eine Benzylgruppe oder eine Alkyl- oder Alkoxyalkylgruppe mit einer Carbonylgruppe und mit 2 bis 5 Kohlenstoffatomen darstellt; $R_2$ ein Wasserstoffatom oder eine Alkylgruppe mit 1 bis 3 Kohlenstoffatomen darstellt; $R_3$ eine Alkylgruppe mit 1 bis 3 Kohlenstoffatomen, die 1 bis 6 Fluoratome enthält, oder eine Gruppe -O-$R_4$ darstellt, worin $R_4$ ein Wasserstoffatom, eine Alkylgruppe mit 1 bis 8 Kohlenstoffatomen, oder eine Benzylgruppe ist; und X ein Halogenatom oder ein Wasserstoffatom darstellt;
worin dieses Derivat der Formel (I) mit einem geeigneten Träger in Kontakt gebracht wird, so daß man eine Fungizid-Zusammensetzung erhält, die den Wirkstoff in einer Konzentration von 0.1 bis 50 Gewichtsprozent enthält.

**7.** Verwendung eines durch die Formel (I)

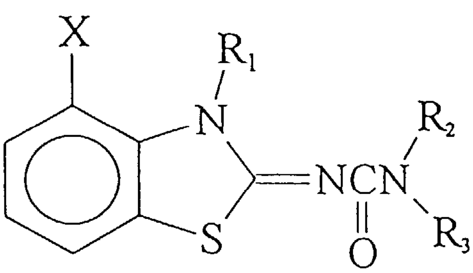

(I)

dargestellten Benzothiazol-Derivats,
worin $R_1$ Alkyl mit 1 bis 8 Kohlenstoffatomen, Alkenyl mit 2 bis 6 Kohlenstoffatomen, Alkinyl mit 2 bis 6 Kohlenstoffatomen, Cycloalkyl mit 3 bis 6 Kohlenstoffatomen, eine Alkoxyalkylgruppe mit 2 bis 5 Kohlenstoffatomen, eine Benzylgruppe oder eine Alkyl- oder Alkoxyalkylgruppe mit einer Carbonylgruppe und mit 2 bis 5 Kohlenstoffatomen darstellt; $R_2$ ein Wasserstoffatom oder eine Alkylgruppe mit 1 bis 3 Kohlenstoffatomen darstellt; $R_3$ eine Alkylgruppe mit 1 bis 3 Kohlenstoffatomen, die 1 bis 6 Fluoratome enthält, oder eine Gruppe -O-$R_4$ darstellt, worin $R_4$ ein Wasserstoffatom, eine Alkylgruppe mit 1 bis 8 Kohlenstoffatomen, oder eine Benzylgruppe ist; und X ein Halogenatom oder ein Wasserstoffatom darstellt,
als Wirkstoff in einer Fungizid-Zusammensetzung für Landwirtschaft und Gartenbau.

**8.** Verwendung nach Anspruch 7, worin die Fungizid-Zusammensetzung wirksam gegen echten Weizenmehltau und Reisbräune ist.

## Revendications

**1.** Dérivé de benzothiazole représenté par la formule (I):

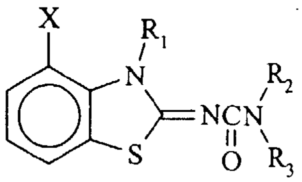

(I)

dans laquelle $R_1$ représente un groupe alkyle de 1 à 8 atomes de carbone, un groupe alcényle de 2 à 6 atomes de carbone, un groupe alcynyle de 2 à 6 atomes de carbone, un groupe cycloalkyle de 3 à 6 atomes de carbone, un groupe alcoxyalkyle de 2 à 5 atomes de carbone, un groupe benzyle, ou un groupe alkyle ou alcoxyalkyle contenant un groupe carbonyle et ayant 2 à 5 atomes de carbone; $R_2$ représente un atome d'hydrogène ou un groupe alkyle de 1 à 3 atomes de carbone; $R_3$ représente un groupe alkyle de 1 à 3 atomes de carbone et contenant 1 à 6 atomes de fluor, ou un groupe représenté par $-O-R_4$ où $R_4$ représente un atome d'hydrogène, un groupe alkyle de 1 à 8 atomes de carbone ou un groupe benzyle; et X représente un atome d'halogène ou un atome d'hydrogène.

**2.** Dérivé de benzothiazole selon la revendication 1, qui est choisi parmi le 3-méthyl-2-N-méthoxycarbamoyliminobenzothiazole, le 3-éthyl-2-N-méthoxycarbamoyliminobenzothiazole, le 3-éthyl-2-N-éthoxycarbamoyliminobenzothiazole, le 3-n-propyl-2-N-méthoxycarbarmoyliminobenzothiazole, le 3-i-propyl-2-N-méthoxycarbamoyliminobenzothiazole, le 3-allyl-2-N-méthoxycarbamoyliminobenzothiazole, le 3-allyl-2-N-méthyl-N-méthoxycarbamoyliminobenzothiazole, le 3-allyl-2-N-éthoxycarbarmoyliminobenzothiazole, le 3-allyl-2-N-n-propoxycarbamoyliminobenzothiazole, le 3-allyl-2-N-(2-propoxy)carbamoyliminobenzothiazole, le 3-allyl-2-N-n-butoxycarbamoyliminobenzothiazole, le 3-allyl-2-N-benzyloxycarbamoyliminobenzothiazole, le 3-allyl-2-N-hydroxycarbamoyliminobenzothiazole, le 3-propynyl-2-N-méthoxycarbamoyliminobenzothiazole, le 3-n-butyl-2-N-méthoxycarbamoyliminobenzothiazole, le 3-i-butyl-2-N-méthoxycarbamoyliminobenzothiazole, le 3-cyclopropylméthyl-2-N-méthoxycarbamoyliminobenzothiazole, le 3-(2-méthyl-2-propényl)-2-N-méthoxycarbamoyliminobenzothiazole, le 3-(2-buténysl)-2-N-méthoxycarbamoyliminobenzothiazole, le 3-cyclopentyl-2-N-méthoxycarbamoyliminobenzothiazole, le 3-n-hexyl-2-N-méthoxycarbamoyliminobenzothiazole, le 3-benzyl-2-N-méthoxycarbamoyliminobenzothiazole, le 3-éthoxycarbonylméthyl-2-N-méthoxycarbamoyliminobenzothiazole, le 3-méthoxyéthyl-2-N-méthoxycarbarmoyliminobenzothiazole, le 3-méthoxyméthyl-2-N-méthoxycarbamoyliminobenzothiazole, le 3-allyl-4-fluoro-2-N-méthoxycarbamoyliminobenzothiazole, le 3-allyl-4-chloro-2-N-méthoxycarbamoyliminobenzothiazole, le 3-cyclopropylméthyl-2-N-(2,2,2-trifluoroéthyl)carbamoyliminobenzothiazole, le 3-benzyl-2-N-(2,2,2-trifluoroéthyl)carbamoyliminobenzothiazole, le 3-i-butyl-2-N-(2,2,2-trifluoroéthyl)carbamoyliminobenzothiazole, le 3-cyclopentyl-2-N-(2,2,2-trifluoroéthyl)carbamoyliminobenzothiazole, le 3-n-hexyl-2-N-(2,2,2-trifluoroéthyl)-carbamoyliminobenzothiazole, le 3-n-butyl-2-N-(2,2,2-trifluoroéthyl)carbamoyliminobenzothiazole, le 3-n-propyl-2-N-(2,2,2-trifluoroéthyl)carbarmoyliminobenzothiazole, le 3-(2-méthyl-2-propényl)-2-N-(2,2,2-trifluoroéthyl)carbamoyliminobenzothiazole, le 3-i-propyl-2-N-(2,2,2-trifluoroéthyl)carbamoyliminobenzothiazole, le 3-éthyl-2-N-(2,2,2-trifluoroéthyl) carbamoyliminobenzothiazole, le 3-allyl-2-N-(2,2,2-trifluoroéthyl)carbamoyliminobenzothiazole, le 3-(2-propynyl)-2-N-(2,2,2-trifluoroéthyl)carbamoyliminobenzothiazole, le 3-allyl-2-N-(2,2,3,3,3-pentafluoropropyl)carbamoyliminobenzothiazole, le 3-allyl-2-N-(2-fluoroéthyl)carbamoyliminobenzothiazole, le 3-éthoxycarbonylméthyl-2-N-(2,2,2-trifluoroéthyl)carbamoyliminobenzothiazole, le 3-carboxyméthyl-2-N-(2,2,2-trifluoroéthyl)carbamoyliminobenzothiazole, le 3-acétylméthyl-2-N-(2,2,2-trifluoroéthyl)carbarmoyliminobenzothiazole, le 3-méthoxyéthyl-2-N-(2,2,2-trifluoroéthyl)carbamoyliminobenzothiazole, le 3-méthoxyméthyl-2-N-(2,2,2-trifluoroéthyl)carbamoyliminobenzothiazole, le 3-allyl-4-fluoro-2-N-(2,2,2-trifluoroéthyl)carbamoyliminobenzothiazole, et le 3-allyl-4-chloro-2-N-(2,2,2-trifluoroéthyl)carbamoyliminobenzothiazole.

**3.** Composition fongicide contenant un dérivé de benzothiazole représenté par la formule (I):

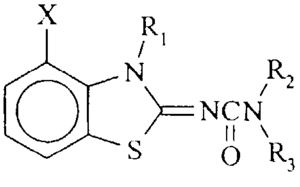

(I)

dans laquelle $R_1$ représente un groupe alkyle de 1 à 8 atomes de carbone, un groupe alcényle de 2 à 6 atomes

de carbone, un groupe alcynyle de 2 à 6 atomes de carbone, un groupe cycloalkyle de 3 à 6 atomes de carbone, un groupe alcoxyalkyle de 2 à 5 atomes de carbone, un groupe benzyle, ou un groupe alkyle ou alcoxyalkyle contenant un groupe carbonyle et ayant 2 à 5 atomes de carbone; $R_2$ représente un atome d'hydrogène ou un groupe alkyle de 1 à 3 atomes de carbone; $R_3$ représente un groupe alkyle de 1 à 3 atomes de carbone et contenant 1 à 6 atomes de fluor, ou un groupe représenté par -O-$R_4$ où $R_4$ représente un atome d'hydrogène, un groupe alkyle de 1 à 8 atomes de carbone ou un groupe benzyle; et X représente un atome d'halogène ou un atome d'hydrogène; et un support.

**4.** Procédé de préparation d'un dérivé de benzothiazole représenté par la formule (I):

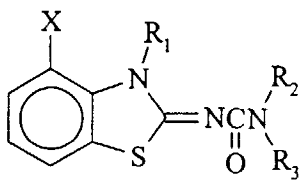

(I)

dans laquelle $R_1$ représente un groupe alkyle de 1 à 8 atomes de carbone, un groupe alcényle de 2 à 6 atomes de carbone, un groupe alcynyle de 2 à 6 atomes de carbone, un groupe cycloalkyle de 3 à 6 atomes de carbone, un groupe alcoxyalkyle de 2 à 5 atomes de carbone, un groupe benzyle, ou un groupe alkyle ou alcoxyalkyle contenant un groupe carbonyle et ayant 2 à 5 atomes de carbone; $R_2$ représente un atome d'hydrogène ou un groupe alkyle de 1 à 3 atomes de carbone; $R_3$ représente un groupe alkyle de 1 à 3 atomes de carbone et contenant 1 à 6 atomes de fluor, ou un groupe représenté par -O-$R_4$ où $R_4$ représente un atome d'hydrogène, un groupe alkyle de 1 à 8 atomes de carbone ou un groupe benzyle; et X représente un atome d'halogène ou un atome d'hydrogène,
qui comprend les étapes suivantes:

(a) on fait réagir un composé représenté par la formule (II) ci-dessous

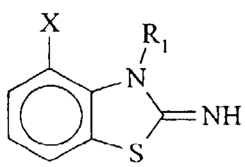

(II)

avec du phosgène dans un solvant approprié choisi dans le groupe constitué par un hydrocarbure aromatique, une cétone, un hydrocarbure halogéné, un ester, un éther et un solvant organique polaire comme le dioxane, en présence d'une base, à une température de 0 à 30°C, pour obtenir un composé de la formule (III) ci-dessous:

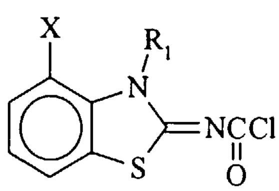

(III)

(b) on fait ensuite réagir le composé correspondant à la formule générale (III) avec une amine de la formule (IV) ci-dessous:

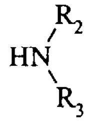

(IV)

à une température de 0 à 30°C pour obtenir le composé (I) désiré.

5. Procédé selon la revendication 4, dans lequel, dans l'étape (a), le solvant est un hydrocarbure halogéné, de préférence le chloroforme ou le chlorure de méthylène, la base est choisie parmi la pyridine, la triéthylamine, l'hydrogénocarbonate de sodium ou l'hydrogénocarbonate de potassium, et la température de la réaction est comprise entre 0 et 5°C, et, dans l'étape (b), la température de la réaction est comprise entre 0 et 5°C.

6. Procédé de préparation d'une composition fongicide contenant un dérivé de benzothiazole représenté par la formule (I):

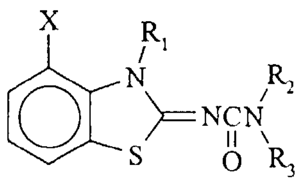

(I)

dans laquelle $R_1$ représente un groupe alkyle de 1 à 8 atomes de carbone, un groupe alcényle de 2 à 6 atomes de carbone, un groupe alcynyle de 2 à 6 atomes de carbone, un groupe cycloalkyle de 3 à 6 atomes de carbone, un groupe alcoxyalkyle de 2 à 5 atomes de carbone, un groupe benzyle, ou un groupe alkyle ou alcoxyalkyle contenant un groupe carbonyle et ayant 2 à 5 atomes de carbone; $R_2$ représente un atome d'hydrogène ou un groupe alkyle de 1 à 3 atomes de carbone; $R_3$ représente un groupe alkyle de 1 à 3 atomes de carbone et contenant 1 à 6 atomes de fluor, ou un groupe représenté par -O-$R_4$ où $R_4$ représente un atome d'hydrogène, un groupe alkyle de 1 à 8 atomes de carbone ou un groupe benzyle; et X représente un atome d'halogène ou un atome d'hydrogène; dans lequel on met ledit dérivé de formule (I) en contact avec un support approprié pour obtenir une composition fongicide contenant l'ingrédient actif à une concentration de 0,1 à 50 % en masse.

7. Utilisation d'un dérivé de benzothiazole représenté par la formule (I):

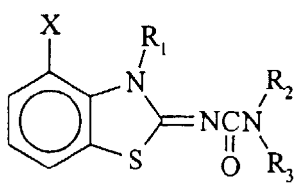

(I)

dans laquelle $R_1$ représente un groupe alkyle de 1 à 8 atomes de carbone, un groupe alcényle de 2 à 6 atomes de carbone, un groupe alcynyle de 2 à 6 atomes de carbone, un groupe cycloalkyle de 3 à 6 atomes de carbone, un groupe alcoxyalkyle de 2 à 5 atomes de carbone, un groupe benzyle, ou un groupe alkyle ou alcoxyalkyle contenant un groupe carbonyle et ayant 2 à 5 atomes de carbone; $R_2$ représente un atome d'hydrogène ou un groupe alkyle de 1 à 3 atomes de carbone; $R_3$ représente un groupe alkyle de 1 à 3 atomes de carbone et contenant 1 à 6 atomes de fluor, ou un groupe représenté par -O-$R_4$ où $R_4$ représente un atome d'hydrogène, un groupe alkyle de 1 à 8 atomes de carbone ou un groupe benzyle; et X représente un atome d'halogène ou un atome d'hydrogène,
comme ingrédient actif dans une composition fongicide pour l'agriculture ou l'horticulture.

8. Utilisation selon la revendication 7, dans laquelle la composition fongicide est efficace contre l'oïdium du blé et la brûlure du riz.